# EUROPEAN PATENT SPECIFICATION

(11) **EP 3 257 945 B1**
(45) Date of publication and mention of the grant of the patent: **28.08.2019**
(21) Application number: 16749435.0
(22) Date of filing: 05.02.2016
(51) Int. Cl.: C12P 7/04, C12N 9/04, C12N 15/10

(54) **RECOMBINANT MICROORGANISM FOR DIOL PRODUCTION**
REKOMBINANTER MIKROORGANISMUS ZUR DIOLHERSTELLUNG
MICRO-ORGANISME DE RECOMBINAISON POUR LA PRODUCTION DE DIOLS

(30) Priority: 09.02.2015 KR 20150019347
(43) Date of publication of application: 20.12.2017
(73) Proprietor: GS Caltex Corporation, Seoul 06141 (KR)
(72) Inventor: PARK, Jong-Myoung, Sejong 30130 (KR); RATHNASI, Chelladurai, Daejeon 34139 (KR)
(74) Representative: Wynne-Jones IP Limited
(86) International application number: PCT/KR2016/001309
(87) International publication number: WO 2016/129895

(56) References cited:
- WO-A2-2010/030711
- KR-A- 20090 025 902
- KR-A- 20140 015 086
- KR-A- 20140 114 726
- KR-A- 20150 010 904
- US-A1- 2012 329 113
- HARRY YIM ET AL: "Metabolic engineering of Escherichia coli for direct production of 1,4-butanediol", NATURE CHEMICAL BIOLOGY, NATURE PUBLISHING GROUP, UNITED KINGDOM, vol. 7, no. 7, 1 July 2011 (2011-07-01), pages 445-452, XP2690293, ISSN: 1552-4469, DOI: 10.1038/NCHEMBIO.580 [retrieved on 2011-05-22]
- WANG QINGZHAO ET AL: "Evolution of D-lactate dehydrogenase activity from glycerol dehydrogenase and its utility for D-lactate production from lignocellulose.", PROCEEDINGS OF THE NATIONAL ACADEMY OF SCIENCES OF THE UNITED STATES OF AMERICA 22 NOV 2011, vol. 108, no. 47, 22 November 2011 (2011-11-22), pages 18920-18925, XP002775005, ISSN: 1091-6490
- GUO XUEWU ET AL: "Effect of the inactivation of lactate dehydrogenase, ethanol dehydrogenase, and phosphotransacetylase on 2,3-butanediol production in Klebsiella pneumoniae strain.", BIOTECHNOLOGY FOR BIOFUELS 26 MAR 2014, vol. 7, no. 1, 26 March 2014 (2014-03-26), page 44, XP021183638,
- YANG TAOWEI ET AL: "Improved Production of 2,3-Butanediol in Bacillus amyloliquefaciens by Over-Expression of Glyceraldehyde-3-Phosphate Dehydrogenase and 2,3-butanediol Dehydrogenase", PLOS ONE, vol. 8, no. 10, October 2013 (2013-10), XP55418804,
- JUNG, MOO - YOUNG ET AL.: 'Improvement of 2, 3-butanedlol Yield in Klebsiella Pneumoniae by Deletion of the Pyruvate Formate-lyase Gene' APPLIED AND ENVIRONMENTAL MICROBIOLOGY vol. 80, no. 19, 2014, pages 6195 - 6203, XP055406522

## Description

### [Technical Field]

The present disclosure relates to a recombinant microorganism for producing a diol.

### [Background Art]

2,3-butanediol containing four carbons and two hydroxyl (-OH) groups is an industrially highly potential chemical that can be used as a precursor for 1,3-butadiene, which is the major constituent of synthetic rubbers, and for methyl ethyl ketone (MEK) which is a solvent. It can also be used directly as an antifreeze agent due to its significant low freezing point and as an octane booster in combination with existing gasoline due to its high octane number.

1,3-propanediol containing three carbons and two hydroxyl (-OH) groups can be used as a monomer for a polymer such as polyester, polyurethane, and the like, and also as an additive for improving the properties of cosmetics and personal hygiene products. In particular, polytrimethylene terephthalate (PTT), a linear aromatic polyester produced by polycondensation of 1,3-propanediol with terephthalic acid, has a unique twist (also called as 'kink' on semi-crystal molecular structures) on the polymer chain and is excellent in rebound resilience and morphostasis. Due to such structural properties, it can be applied to a wide variety of fields such as fibers, packaging and films, nonwoven fabrics, and engineering plastics.

The diols can be produced either by a chemical synthesis process or microbial fermentation process. However, the chemical synthesis process may cause environmental pollutants in the process and high synthesis costs. The microbial fermentation process may also produce succinate, acetate and the like as well as diols (such as 2,3-butanediol and 1,3-propanediol) in the fermentation culture, resulting in an increase in separation and recovery costs.

The inventors have endeavored to develop a recombinant microorganism capable of producing a diol at industrial levels and completed the present invention by preparing a recombinant microorganism having high diol-producing ability while having low producing ability of succinate and acetate.

### [Disclosure]

### [Technical Problem]

It is an object of the present disclosure to provide a recombinant microorganism for producing a diol, which has high diol-producing ability while having low producing ability of byproducts.

### [Technical Solution]

In accordance with one aspect of the present disclosure, there is provided a recombinant microorganism for producing 2,3-butanediol and 1,3-propanediol, wherein a pathway for converting pyruvate to lactate and a pathway for converting oxaloacetate to succinate are inhibited in a microorganism having a diol biosynthetic pathway wherein the microorganism is *Klebsiella* and the recombinant microorganism has decreased succinate-producing ability in comparison to a wild type microorganism.

In accordance with another aspect of the present disclosure, there is provided a method for producing a diol using the recombinant microorganism.

### [Advantageous Effects]

The recombinant microorganism of the present disclosure not only has a high diol-producing ability, but also has a low producing ability of byproducts, enabling easy production and recovery of a diol.

### [Description of Drawings]

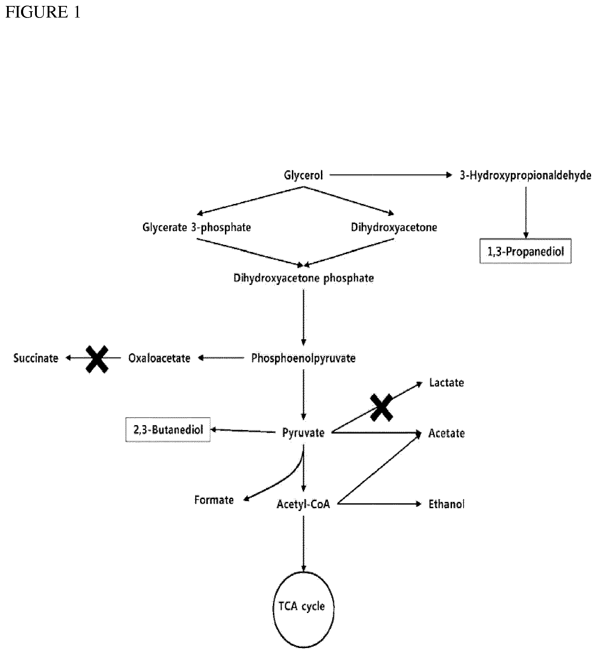

Figure 1 shows the change in metabolic pathway through gene deletion in the recombinant microorganism of the present disclosure.
Figure 2 shows the diol-producing ability and cell concentration (OD600 value) of Kp wt.
Figure 3 shows the byproduct-producing ability in the fermentation culture of Kp wt.
Figure 4 shows the diol-producing ability and cell concentration (OD600 value) of Kp ΔldhA.
Figure 5 shows the byproduct-producing ability in the fermentation culture of Kp ΔldhA.
Figure 6 shows the diol-producing ability and cell concentration (OD600 value) of Kp ΔldhA Δmdh.
Figure 7 shows the byproduct-producing ability in the fermentation culture of Kp ΔldhA Δmdh.
Figure 8 shows the diol-producing ability and cell concentration (OD600 value) of Kp ΔldhA ΔfumA.
Figure 9 shows the byproduct-producing ability in the fermentation culture of Kp ΔldhA ΔfumA.
Figure 10 shows the diol-producing ability of the strains over time.
Figure 11 shows the succinate-producing ability of the strains over time.

### [Best Mode]

The present disclosure is directed to a recombinant microorganism for producing 2,3-butanediol and 1,3-propanediol, wherein a pathway for converting pyruvate to lactate and a pathway for converting oxaloacetate to succinate are inhibited in a microorganism having a diol biosynthetic pathway wherein the microorganism is *Klebsiella* and the recombinant microorganism has decreased succinate-producing ability in comparison to a wild type microorganism.

The present disclosure is also directed to a method for producing a diol, comprising culturing the recombinant microorganism of the present disclosure, and recovering a diol from the culture.

Hereinafter, the present invention will be described in detail.

### Microorganisms having a diol biosynthetic pathway

The microorganism of the present disclosure has a diol biosynthetic pathway. The diol biosynthetic pathway refers to a pathway in which a diol is synthesized from a particular metabolite in a microorganism. The diol biosynthetic pathway of the present disclosure may be a pathway in which 2,3-butanediol is synthesized from pyruvate and/or a pathway in which 1,3-propanediol is synthesized from glycerol. Preferably, the microorganism of the present disclosure has a pathway for biosynthesizing a diol from a carbon source such as glycerol.

In addition, the microorganism of the present disclosure may be a recombinant microorganism having a diol biosynthetic pathway by gene recombination. Preferably, the microorganism is a microorganism having the ability to produce 1,3-propanediol and 2,3-butanediol. Preferably, the microorganism is *Klebsiella sp*., and more preferably *Klebsiella pneumoniae.*

### Pathway for converting pyruvate to lactate

Lactate dehydrogenase catalyzes the conversion of pyruvate to lactate. The inhibition of the lactate dehydrogenase can inhibit the pathway for converting pyruvate to lactate. It may be achieved by inhibition of expression of the lactate dehydrogenase, inhibition of activity of the lactate dehydrogenase, and the like. For example, the lactate dehydrogenase may be inhibited by a suitable method known in the art, such as deletion of *ldhA* which is a gene encoding the lactate dehydrogenase, mutagenesis of the gene (*e.g*., mutation of inhibiting expression of a normal gene such as variations, substitutions, deletion or insertion of some bases), regulation of gene expression in a transcription or translation process, and the like.

### Pathway for converting oxaloacetate to succinate

The microorganism having the pyruvate biosynthesis pathway of the present disclosure has a pathway for converting oxaloacetate to succinate. Preferably, the pathway for converting oxaloacetate to succinate is a pathway in which oxaloacetate is converted to malate and fumarate and then to succinate.

Preferably, the pathway for converting oxaloacetate to succinate is inhibited by inhibiting a pathway for converting oxaloacetate to maleate or a pathway for converting malate to fumarate, more preferably by inhibiting malate dehydrogenase or fumarase. Even more preferably, the inhibition of the pathway for converting oxaloacetate to succinate is performed by deletion of the *mdh* or *fumA* (or *fumABC*) gene, mutagenesis of the gene (*e.g*., mutation of inhibiting expression of a normal gene such as variations, substitutions, deletion or insertion of some bases), regulation of gene expression in a transcription or translation process, and the like. The *mdh* gene has a nucleotide sequence having 80% or more homology with SEQ ID NO: 9, preferably 85% or more homology, more preferably 90% or more homology, and even more preferably 95% or more homology. The *fumA* gene has a nucleotide sequence having 80% or more homology with SEQ ID NO: 17, preferably 85% or more homology, more preferably 90% or more homology, and even more preferably 95% or more homology.

### Diol

The recombinant microorganism of the present disclosure is one for producing a diol. The recombinant microorganism of the present disclosure produces, among fermentation products, 1,3-propanediol and 2,3-butanediol in a high yield.

### Recombinant microorganism for producing a diol

The recombinant microorganism of the present disclosure exhibits high productivity of a diol (amount of diol produced per unit volume per unit time), high producing ability (amount of diol produced per unit volume, *i.e*., concentration of diol in the fermentation liquid) and high yield (production of diol relative to carbon sources). In particular, the recombinant microorganism of the present disclosure is characterized by having a higher concentration of diol in the fermentation liquid than the wild-type microorganism upon fermentation. In addition, the recombinant microorganism of the present disclosure is characterized in that the production of oxidation byproducts such as lactate, succinate, acetate and the like is inhibited.

Preferably, the recombinant microorganism of the present disclosure has increased productivity of diol but inhibited productivity of succinate (*i.e*., succinic acid) or acetate (i.e., acetic acid) as compared with wild-type microorganisms. More preferably, the recombinant microorganism of the present disclosure has the diol productivity of at least 15 times the succinate productivity, more preferably the diol productivity of at least 18 times the succinate productivity. Particularly, among the recombinant microorganisms of the present disclosure, the recombinant microorganism in which the pathway for converting oxaloacetate to malate is inhibited has the diol productivity of at least 80 times the succinate productivity, preferably the diol productivity of at least 90 times the succinate productivity, and more preferably the diol productivity of at least 100 times the succinate productivity.

More preferably, the recombinant microorganism of the present disclosure has the inhibited ethanol-producing ability or the succinate-producing ability, as compared to a recombinant microorganism in which the pathway for converting pyruvate to lactate is inhibited and the pathway for converting oxaloacetate to succinate is not inhibited

### Method for producing diol

The present disclosure relates to a method for producing a diol, comprising culturing the recombinant microorganism of the present disclosure, and recovering a diol from the culture. The culture is carried out under aerobic conditions, preferably under micro-aerobic conditions. For example, the culture is carried out while supplying oxygen, *i.e*., air, during the culture, and specifically, it may be carried out, without limitation, by stirring.

Various advantages and features of the present invention and methods accomplishing thereof will become apparent from the following description of embodiments. However, the present invention is not limited to exemplary embodiment disclosed herein but will be implemented in various forms. The exemplary embodiments are provided by way of example only so that a person of ordinary skilled in the art can fully understand the disclosures of the present invention and the scope of the present invention. Therefore, the present invention will be defined only by the scope of the appended claims.

### <Materials and methods>

*Klebsiella pneumoniae* GSC123 (KCTC12133BP) was used.

The metabolite-producing ability of the microorganism of the present disclosure was calculated as follows.
- Concentration of product (g/L): Amount of specific product produced per unit volume
- Yield of product (g/g): production of specific product (g) / carbon source (g)
- Productivity of product (g/L/h): amount of specific product produced per unit volume per unit time

### Experimental Example 1: Preparation of a recombinant microorganism

### Klebsiella pneumoniae GSC123 ΔldhA (Kp ΔldhA) strain

A *Klebsiella pneumoniae* GSC123 ΔldhA (Kp ΔldhA) strain having lactate dehydrogenase (ldhA) deleted was prepared as follows. First, in order to clone the lactate dehydrogenase of *Klebsiella pneumoniae,* a homologous region 1 (SEQ ID NO: 2) of *ldhA* (SEQ ID NO: 1) as a target gene, was amplified by PCR using primers of SEQ ID NOs: 3 and 4. In addition, a homologous region 2 (SEQ ID NO: 5) was amplified by PCR using primers of SEQ ID NOs: 6 and 7. Then, both of the homologous regions 1 and 2 as a template were simultaneously amplified by PCR to obtain a DNA fragment (SEQ ID NO: 8) having the homologous regions 1 and 2 ligated (see Table 1).

In order to increase the recombination probability of the target gene, the resulting DNA fragment may include an antibiotic resistance gene and the like. Further, the DNA fragment may include a *sacB* gene encoding a levansucrase enzyme to remove the recombinant antibiotic resistance gene in the chromosome.

The prepared DNA fragment was transformed into a wild-type *Klebsiella pneumoniae* (*i.e*., *Klebsiella pneumoniae* GSC123 (KCTC12133BP)) using electroporation (25 uF, 200 Ω, 18 kV/cm), and the target gene was capable of being removed using a homologous recombination mechanism of the microorganism.

**TABLE 1**

| SEQ ID | Sequence |
|---|---|
| 1 | |
| 2 | |
| | |
| 3 | Kp_IdhA_FP1 - TAGAGGATCCCAAGCGTGCGCGGTGAACCG |
| 4 | Kp_ldhA_RP1 - GAGGAGCACAAAAGGGAAAGGCGAAGACTTTTCTCCAGTGATTATAC |
| 5 | |
| 6 | Kp_IdhA_ FP2 - GTATAATCACTGGAGAAAAGTCTTCGCCTTTCCCTTTTGTGCTCCTC |
| 7 | Kp_ IdhA_ RP2 - ATCGCGGCCGCGCGTGAAACCGCGACGCGCC |
| 8 | |
| | |

### Klebsiella pneumoniae GSC123 ΔldhA Δmdh (Kp ΔldhA Δmdh) strain

A *Klebsiella pneumoniae* GSC123 ΔldhA Δmdh (Kp ΔldhA Δmdh) strain having malate dehydrogenase (mdh) further deleted was prepared as follows. First, in order to clone the malate dehydrogenase of *Klebsiella pneumoniae,* a homologous region 1 (SEQ ID NO: 10) of *mdh* (SEQ ID NO: 9) as a target gene, was amplified by PCR using primers of SEQ ID NOs: 11 and 12. In addition, a homologous region 2 (SEQ ID NO: 13) was amplified by PCR using primers of SEQ ID NOs: 14 and 15. Then, both of the homologous regions 1 and 2 as a template were simultaneously amplified by PCR to obtain a DNA fragment (SEQ ID NO: 16) having the homologous regions 1 and 2 ligated (see Table 2).

In order to increase the recombination probability of the target gene, the resulting DNA fragment may include an antibiotic resistance gene and the like. Further, the DNA fragment may include a *sacB* gene encoding a levansucrase enzyme to remove the recombinant antibiotic resistance gene in the chromosome.

The prepared DNA fragment was transformed into the *Klebsiella pneumoniae* GSC123 ΔldhA (Kp ΔldhA) strain having lactate dehydrogenase (ldhA) deleted using electroporation (25 uF, 200 Ω, 18 kV/cm), and the target gene was capable of being removed using a homologous recombination mechanism of the microorganism.

**TABLE 2**

| SEQ ID | Sequence |
|---|---|
| 9 | |
| 10 | |
| 11 | mdh_FP1 - GTCGACCGCGTTAGTAAAAGAACGTATAGACGG |
| 12 | mdh_RP1 - GCTCTTCTGTTAGGGCGAGACCTAAACTCCTTATTATGG |
| 13 | |
| 14 | mdh_FP7 - CCATAATAAGGAGTTTAGGTCTCGCCCTAACAGAAGAGC |
| 15 | mdh_RP2 - GCGGCCGCTGAACAGCACCAGCCATAATCAGCTG |
| 16 | |
| | |
| | |

### Klebsiella pneumoniae GSC123 ΔldhA ΔfumA (Kp ΔldhA ΔfumA) strain

A *Klebsiella pneumoniae* GSC123 ΔldhA ΔfumA (Kp ΔldhA Δ fumA) strain having fumarase (fumA) further deleted was prepared as follows. First, in order to clone the fumarase of *Klebsiella pneumoniae,* a homologous region 1 (SEQ ID NO: 18) of *fumA* (SEQ ID NO: 17) as a target gene, was amplified by PCR using primers of SEQ ID NOs: 19 and 20. In addition, a homologous region 2 (SEQ ID NO: 21) was amplified by PCR using primers of SEQ ID NOs: 22 and 23. Then, both of the homologous regions 1 and 2 as a template were simultaneously amplified by PCR to obtain a DNA fragment (SEQ ID NO: 24) having the homologous regions 1 and 2 ligated (see Table 3).

In order to increase the recombination probability of the target gene, the resulting DNA fragment may include an antibiotic resistance gene and the like, and may include a *sacB* gene encoding a levansucrase enzyme to remove the antibiotic resistance gene recombined in the chromosome.

The prepared DNA fragment was transformed into the *Klebsiella pneumoniae* GSC123 ΔldhA (Kp ΔldhA) strain having lactate dehydrogenase (ldhA) deleted using electroporation (25 uF, 200 Ω, 18 kV/cm), and the target gene was capable of being removed using a homologous recombination mechanism of the microorganism.

**TABLE 3**

| SEQ ID | Sequence |
|---|---|
| 17 | |
| 18 | |
| | |
| 19 | fumA FP1 - GGATCCTTTCAGCAAACAGGAACATCGCTTCGCCTGGG |
| 20 | fumA_ RP1 - CTAACGAAAACAGGTTTGTCATCTGTACTCTCACTTACTGCTTTG |
| 21 | |
| 22 | fumA_FP2 CAAAGCAGTAAGTGAGAGTACAGATGACAAACCTGTTTTCGTTAG |
| 23 | fumA_RP2 - GCGGCCGCTTCGAATTTGTTCGGCGCGGTGACAAATG |
| 24 | |
| | |

### Experimental Example 2: Production of a diol

The recombinant strains prepared in Experimental Example 1 were cultured to produce a diol. As a comparative example, a wild-type *Klebsiella pneumoniae* GSC123 (Kp wt) was used.

Each of the recombinant strains was inoculated into 300 ml of complex medium and cultured at 37°C for 16 hours. Then, the culture was inoculated into 3 L of complex medium and fermented. The conditions for fermentation were as follows: micro-aerobic condition (an aerobic speed of 0.5 vvm and a stirring speed of 300 rpm), 20 g/L glycerol at the start, 30 g/L glycerol at the feeding, pH 6.0, and a culture temperature of 37 °C of Ammonia water (NH₄OH) was used to adjust pH during fermentation. Samples were taken during the fermentation of the recombinant *Klebsiella* strains, and the growth rates were determined by measuring optical density 600 (OD600) of the taken samples. Then, the taken samples were centrifuged at 13,000 rpm for 10 minutes, and the supernatant was analyzed for the concentration of metabolites by liquid chromatography (HPLC).

As a result, the recombinant strain having ldhA deleted (Kp ΔldhA) exhibited a dramatic decrease in lactate, which was the greatest byproduct of a wild-type *Klebsiella pneumoniae* (Kp wt). However, other byproducts such as ethanol, succinate and the like were increased, and in particular the content of succinate was about 10% based on diol.

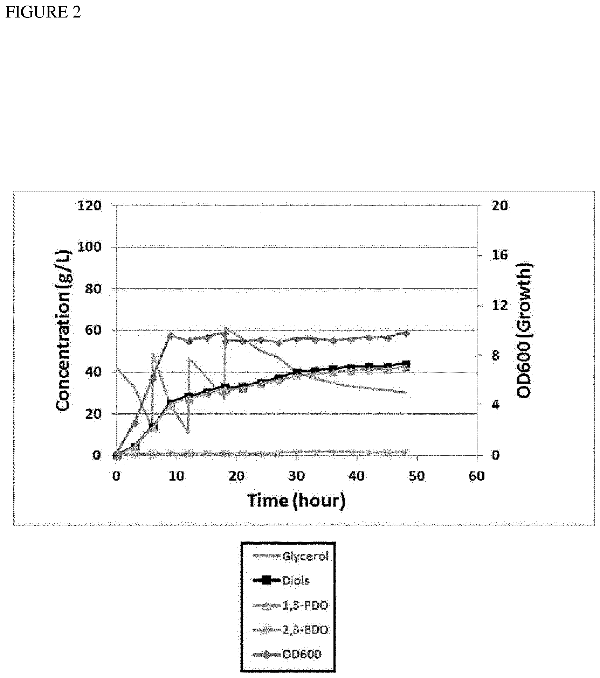

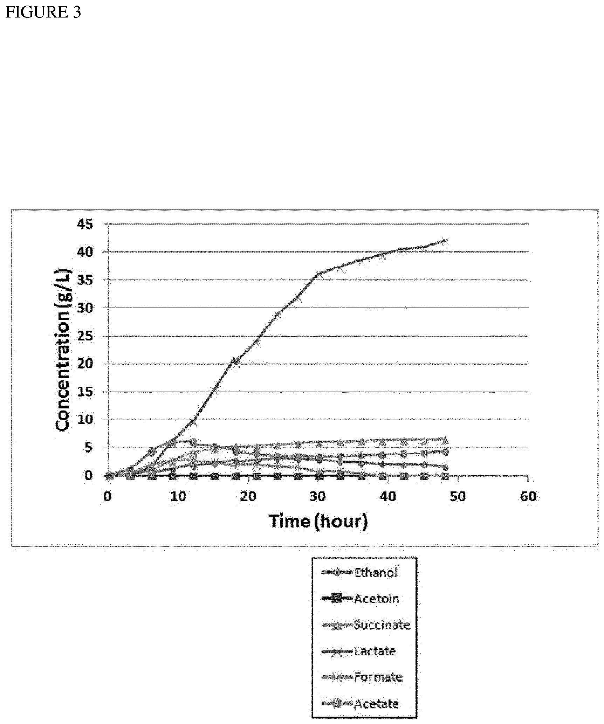

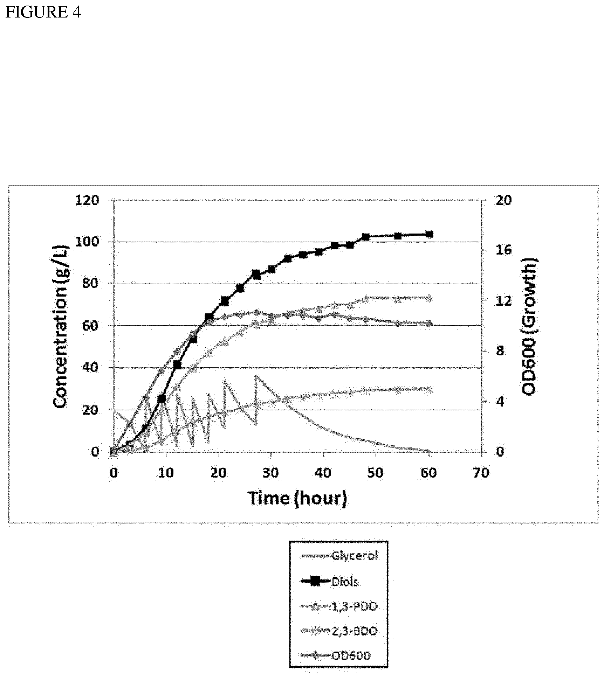

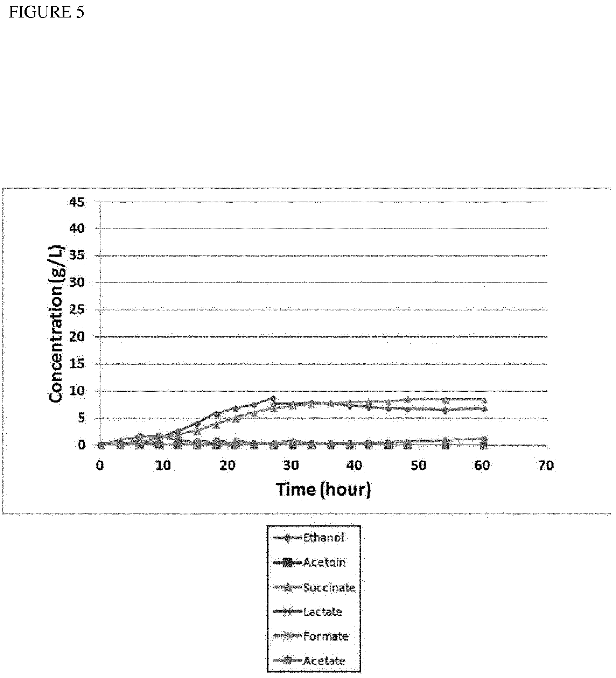

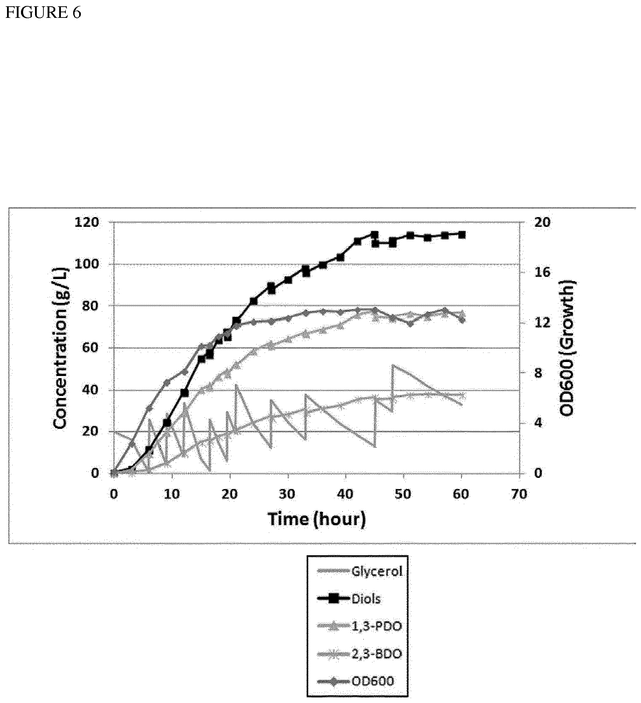

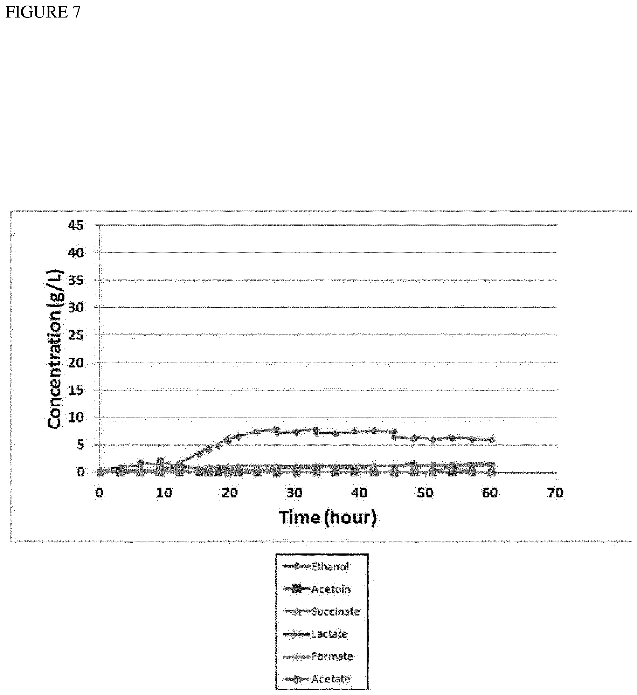

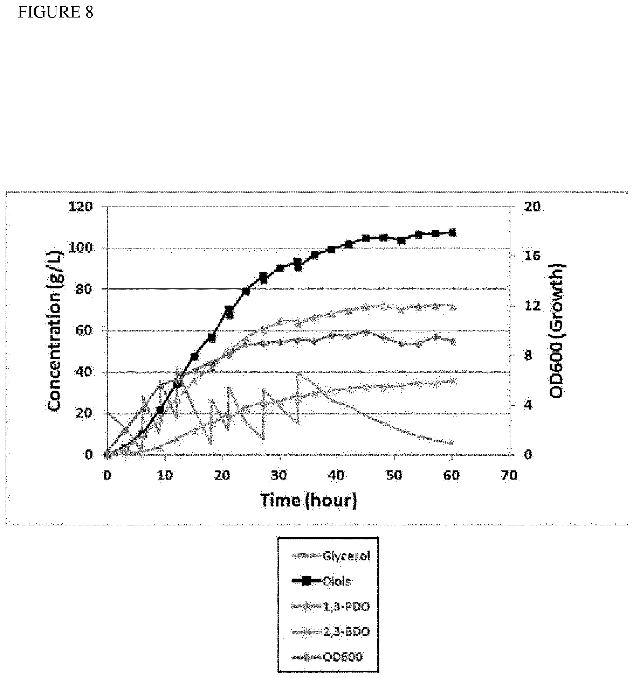

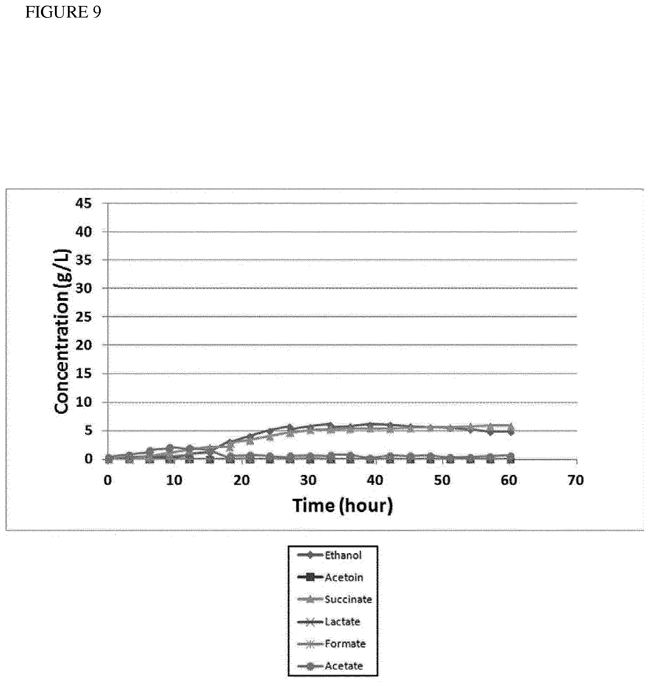

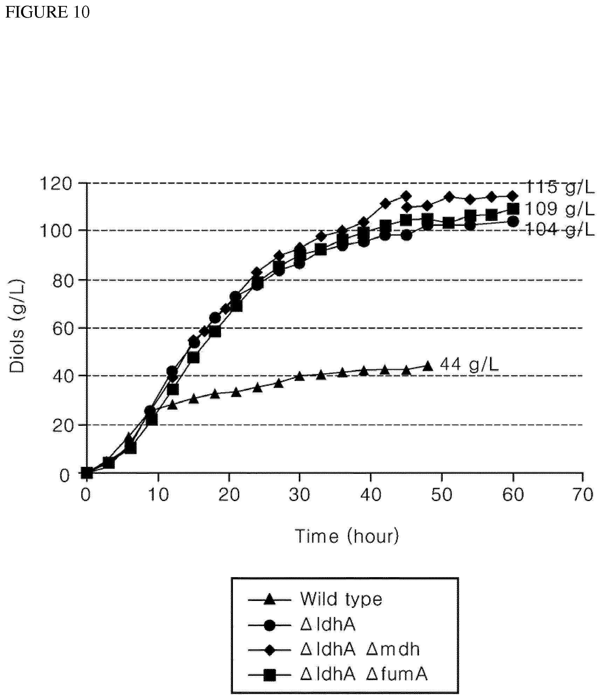

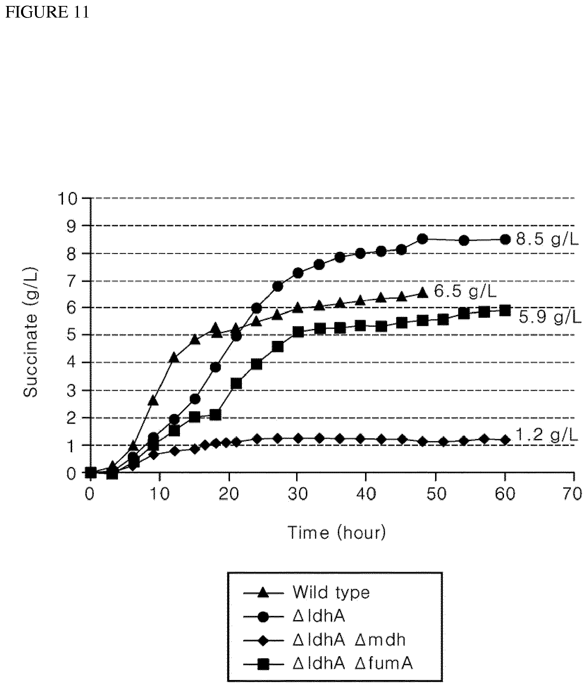

On the other hand, it was found that the recombinant strain having both of ldhA and mdh deleted (Kp ΔldhA Δmdh) and the recombinant strain having both of ldhA and fumA deleted (Kp ΔldhA ΔfumA) exhibited increased diol production and significantly reduced acetate and succinate production, as compared with a wild-type strain and the recombinant strain having ldhA deleted (Kp ΔldhA). In particular, the recombinant strain having both of ldhA and mdh deleted (Kp ΔldhA Δmdh) and the recombinant strain having both of ldhA and fumA deleted (Kp ΔldhA ΔfumA) produced about 10% and 5% higher diols and about 85% and 31% lower succinate than the recombinant strain having ldhA deleted (Kp ΔldhA), respectively. It was also found that the recombinant strain having both of ldhA and mdh deleted (Kp ΔldhA Δmdh) and the recombinant strain having both of ldhA and fumA deleted (Kp ΔldhA ΔfumA) showed a decrease in acetate and ethanol production (see Tables 4-5 and Figs. 2-11; Figure 2 shows the diol-producing ability and cell concentration (OD600 value) of Kp wt; Figure 3 shows the byproduct-producing ability in the fermentation culture; Figure 4 shows the diol-producing ability and cell concentration (OD600 value) of Kp ΔldhA; Figure 5 shows the byproduct-producing ability in the fermentation culture; Figure 6 shows the diol-producing ability and cell concentration (OD600 value) of Kp ΔldhA Δmdh; Figure 7 shows byproduct-producing ability in the fermentation culture; Figure 8 shows the diol-producing ability and cell concentration (OD600 value) of Kp ΔldhA ΔfumA; Figure 9 shows the byproduct-producing ability in the fermentation culture; Figure 10 shows the diol-producing ability of the strains over time; and Figure 11 shows the succinate-producing ability of the strains over time).

**TABLE 4**

| | Wild-type (Kp wt) | Kp ΔldhA | Kp ΔldhA Δmdh | Kp ΔldhA ΔfumA |
|---|---|---|---|---|
| Yield of diol (g/g) | 0.4 | 0.6 | 0.63 | 0.61 |
| Productivity of diol (g/L/h) | 0.9 | 2.0 | 2.2 | 2.1 |
| Productivity of succinate (g/L/h) | 0.14 | 0.14 | 0.02 | 0.10 |

**TABLE 5**

| Strain | Fermentation | | | | product | | |
|---|---|---|---|---|---|---|---|
| | 1,3-propanediol | succinate | lactate | formate | 2,3-butanediol | ethanol | acetate |
| Wild-type (Kp wt | 42.3 | 6.5 | 42.1 | 0.05 | 1.7 | 1.7 | 4.4 |
| Kp ΔldhA | 73.7 | 8.5 | 0 | 0 | 30.2 | 6.7 | 1.2 |
| Kp ΔldhA Δmdh | 77.3 | 1.2 | 0 | 0.1 | 37.6 | 5.9 | 1.6 |
| Kp ΔldhA ΔfumA | 73.1 | 5.9 | 0 | 0 | 35.9 | 4.8 | 0.6 |

### <Accession Number>

Depositary Authority: Korea Research Institute of Bioscience and Biotechnology
Accession Number: KCTC12133BP
Date of deposit: 2012-02-16

### [Industrial Applicability]

The present disclosure provides a recombinant microorganism capable of producing a diol at industrial levels.

### [SEQUENCE LISTING FREE TEXT]

### Description on Sequence Number

SEQ ID NO: 1: nucleotide sequence of *ldhA,* a lactate dehydrogenase gene.
SEQ ID NO: 2: nucleotide sequence of the homologous region 1 of *ldhA.*
SEQ ID NO: 3: primer for PCR amplification of the homologous region 1 of *ldhA.*
SEQ ID NO: 4: primer for PCR amplification of the homologous region 1 of *ldhA.*
SEQ ID NO: 5: nucleotide sequence of the homology region 2 of *ldhA.*
SEQ ID NO: 6: primer for PCR amplification of the homologous region 2 of *ldhA.*
SEQ ID NO: 7: primer for PCR amplification of the homologous region 2 of *ldhA.*
SEQ ID NO: 8: DNA fragment in which the homologous regions 1 and 2 of *ldhA* are ligated, prepared by PCR amplification using the homologous regions 1 and 2 as a template.
SEQ ID NO: 9: nucleotide sequence of *mdh*, a malate dehydrogenase gene.
SEQ ID NO: 10: nucleotide sequence of the homologous region 1 of *mdh*.
SEQ ID NO: 11: primer for PCR amplification of the homologous region 1 of *mdh.*
SEQ ID NO: 12: primer for PCR amplification of the homologous region 1 of *mdh.*
SEQ ID NO: 13: nucleotide sequence of the homologous region 2 of *mdh.*
SEQ ID NO: 14: primer for PCR amplification of the homologous region 2 of *mdh.*
SEQ ID NO: 15: primer for PCR amplification of the homologous region 2 of *mdh.*
SEQ ID NO: 16: DNA fragment in which the homologous regions 1 and 2 of *mdh* are ligated, prepared by PCR amplification using the homologous regions 1 and 2 as a template.
SEQ ID NO: 17: nucleotide sequence of *fumA*, a fumarase gene.
SEQ ID NO: 18: nucleotide sequence of the homologous region 1 of *fumA.*
SEQ ID NO: 19: primer for PCR amplification of the homologous region 1 of *fumA.*
SEQ ID NO: 20: primer for PCR amplification of the homologous region 1 of *fumA.*
SEQ ID NO: 21: nucleotide sequence of the homologous region 2 of *fumA.*
SEQ ID NO: 22: primer for PCR amplification of the homologous region 2 of *fumA.*
SEQ ID NO: 23: primer for PCR amplification of the homologous region 2 of *fumA.*
SEQ ID NO: 24: DNA fragment in which the homologous regions 1 and 2 of *fumA* are ligated, prepared by PCR amplification using the homologous regions 1 and 2 as a template.

## Claims

1. A recombinant microorganism for producing 2,3-butanediol and 1,3-propanediol, wherein a pathway for converting pyruvate to lactate and a pathway for converting oxaloacetate to succinate are inhibited in a microorganism having a diol biosynthetic pathway wherein the microorganism is *Klebsiella* and the recombinant microorganism has decreased succinate-producing ability in comparison to a wild type microorganism.

2. The recombinant microorganism of claim 1, wherein the pathway for converting pyruvate to lactate is a pathway in which the conversion of pyruvate to lactate is controlled by lactate dehydrogenase.

3. The recombinant microorganism of claim 1, wherein the pathway for converting oxaloacetate to succinate is a pathway in which oxaloacetate is converted to malate and fumarate, and then to succinate.

4. The recombinant microorganism of claim 1, wherein the inhibition of the pathway for converting oxaloacetate to succinate is performed by inhibiting a pathway for converting oxaloacetate to malate or a pathway for converting malate to fumarate.

5. The recombinant microorganism of claim 1, wherein the 2,3-butanediol producing ability and 1,3-propanediol -producing ability is increased.

6. The recombinant microorganism of claim 1, wherein the 2,3-butanediol producing ability and 1,3-propanediol producing ability is increased and an acetate producing ability is decreased.

7. The recombinant microorganism of claim 1, wherein a succinate-producing ability is decreased, as compared to a recombinant microorganism in which the pathway for converting pyruvate to lactate is inhibited and the pathway for converting oxaloacetate to succinate is not inhibited.

8. The recombinant microorganism of claim 1, wherein an ethanol-producing ability is decreased, as compared to a recombinant microorganism in which the pathway for converting pyruvate to lactate is inhibited and the pathway for converting oxaloacetate to succinate is not inhibited.

9. A method for producing a diol, comprising culturing the recombinant microorganism of claim 1, and recovering a diol from the culture.

## Patentansprüche

1. Rekombinanter Mikroorganismus zur Erzeugung von 2,3-Butandiol und 1,3-Propandiol, wobei ein Weg zum Umwandeln von Pyruvat in Laktat und ein Weg zum Umwandeln von Oxaloacetat in Succinat in einem Mikroorganismus gehemmt sind, der einen biosynthetischen Diol-Weg aufweist, wobei der Mikroorganismus *Klebsiella* ist und der rekombinante Mikroorganismus verglichen mit einem Wildtyp-Mikroorganismus ein vermindertes Succinat-Erzeugungsvermögen aufweist.

2. Rekombinanter Mikroorganismus nach Anspruch 1, bei dem der Weg zum Umwandeln von Pyruvat in Laktat ein Weg ist, bei dem die Umwandlung von Pyruvat in Laktat durch Laktatdehydrogenase gesteuert wird.

3. Rekombinanter Mikroorganismus nach Anspruch 1, bei dem der Weg zum Umwandeln von Oxaloacetat in Succinat ein Weg ist, bei dem Oxaloacetat in Malat und Fumarat und dann in Succinat umgewandelt wird.

4. Rekombinanter Mikroorganismus nach Anspruch 1, bei dem die Hemmung des Wegs zum Umwandeln von Oxaloacetat in Succinat durch Hemmen eines Wegs zum Umwandeln von Oxaloacetat in Malat oder eines Wegs zum Umwandeln von Malat in Fumarat durchgeführt wird.

5. Rekombinanter Mikroorganismus nach Anspruch 1, bei dem das Vermögen zur Erzeugung von 2,3-Butandiol und das Vermögen zur Erzeugung von 1,3-Propandiol erhöht sind.

6. Rekombinanter Mikroorganismus nach Anspruch 1, bei dem das Vermögen zur Erzeugung von 2,3-Butandiol und das Vermögen zur Erzeugung von 1,3-Propandiol erhöht sind und ein Vermögen zur Erzeugung von Acetat vermindert ist.

7. Rekombinanter Mikroorganismus nach Anspruch 1, bei dem das Vermögen zur Erzeugung von Succinat verglichen mit einem rekombinanten Mikroorganismus, bei dem der Weg zum Umwandeln von Pyruvat in Laktat gehemmt ist und der Weg zum Umwandeln von Oxaloacetat in Succinat nicht gehemmt ist, vermindert ist.

8. Rekombinanter Mikroorganismus nach Anspruch 1, bei dem das Vermögen zur Erzeugung von Ethanol verglichen mit einem rekombinanten Mikroorganismus, bei dem der Weg zum Umwandeln von Pyruvat in Laktat gehemmt ist und der Weg zum Umwandeln von Oxaloacetat in Succinat nicht gehemmt ist, vermindert ist.

9. Verfahren zur Herstellung eines Diols, welches das Kultivieren des rekombinanten Mikroorganismus von Anspruch 1 und das Gewinnen eines Diols aus der Kultur umfasst.

## Revendications

1. Microorganisme recombinant pour la production de 2,3-butanediol et de 1,3-propanediol, dans lequel une voie de conversion de pyruvate en lactate et une voie de conversion d'oxaloacétate en succinate sont inhibées dans un microorganisme ayant une voie de biosynthèse de diols, dans lequel le microorganisme est *Klebsiella* et le microorganisme recombinant a une aptitude diminuée à la production de succinate par comparaison avec un microorganisme de type sauvage.

2. Microorganisme recombinant selon la revendication 1, dans lequel la voie de conversion de pyruvate en lactate est une voie dans laquelle la conversion de pyruvate en lactate est contrôlée par une lactate déshydrogénase.

3. Microorganisme recombinant selon la revendication 1, dans lequel la voie de conversion d'oxaloacétate en succinate est une voie dans laquelle l'oxaloacétate est converti en malate et fumarate, puis en succinate.

4. Microorganisme recombinant selon la revendication 1, dans lequel l'inhibition de la voie de conversion d'oxaloacétate en succinate est effectuée par inhibition d'une voie de conversion d'oxaloacétate en malate ou d'une voie de conversion de malate en fumarate.

5. Microorganisme recombinant selon la revendication 1, dans lequel l'aptitude à la production de 2,3-butanediol et l'aptitude à la production de 1,3-propanediol sont augmentées.

6. Microorganisme recombinant selon la revendication 1, dans lequel l'aptitude à la production de 2,3-butanediol et l'aptitude à la production de 1,3-propanediol sont augmentées et une aptitude à la production d'acétate est diminuée.

7. Microorganisme recombinant selon la revendication 1, dans lequel une aptitude à la production de succinate est diminuée, par comparaison avec un microorganisme recombinant dans lequel la voie de conversion de pyruvate en lactate est inhibée et la voie de conversion d'oxaloacétate en succinate n'est pas inhibée.

8. Microorganisme recombinant selon la revendication 1, dans lequel une aptitude à la production d'éthanol est diminuée, par comparaison avec un microorganisme recombinant dans lequel la voie de conversion de pyruvate en lactate est inhibée et la voie de conversion d'oxaloacétate en succinate n'est pas inhibée.

9. Procédé de production d'un diol, comprenant la culture du microorganisme recombinant de la revendication 1, et la récupération d'un diol à partir de la culture.


<110> GS CALTEX
<120> RECOMBINANT MICROORGANISM FOR PRODUCING DIOLS
<130> DNp150068
<160> 24
<170> KopatentIn 2.0
<210> 1
   <211> 990
   <212> DNA
   <213> Klebsiella pneumoniae
<400> 1
   atgaaaatcg cggtttatag tacgaagcag tacgataaaa agtacctgca gcacgttaat      60
   gatgcatacg gctttgaact ggaattcttc gatttcctgc tgacagcgaa gactgccaaa     120
   accgccaacg gttgcgaagc ggtatgtatc ttcgtcaatg acgacggcag ccgcccggtg     180
   ctggaagagc tgaaggccca cggggtgaaa tatatcgccc tgcgctgcgc cgggtttaac     240
   aacgtcgacc ttgaggcggc aaaggagctt ggcctgcgcg tcgtgcgcgt tccagcttac     300
   tctccggaag cggtcgctga gcatgcgatc ggtatgatga tgtcgctcaa ccgccgcatc     360
   caccgcgctt accagcgtac ccgcgatgcc aatttctccc tcgaaggcct caccggcttc     420
   accatgtacg gcaaaaccgc cggggtgatc ggcaccggga aaattggcgt agcgatgttg     480
   cggatcctca aaggcttcgg catgcgcctg ctggcgttcg acccgtaccc aagcgccgcc     540
   gcgctggagc tgggggtgga atatgttgac ctcgccacgc tgtacaagga atcggacgtg     600
   atctccctgc actgtccgct gaccgacgaa aactaccacc tgctcaatcg cgaagctttc     660
   gatcagatga aagacggggt gatggtgatc aacaccagcc gcggcgccct gatcgactct     720
   caggcggcca tcgacgccct gaagcaccag aaaattggcg cgctggggct ggacgtttat     780
   gagaacgaac gcgatctgtt ctttgaagac aaatccaacg acgtgatcca ggacgatgtc     840
   ttccgccgcc tctccgcctg ccataacgtg ctgtttaccg gccaccaggc gttcctcacc     900
   gccgaggcgc tgatcagcat ttcggagacc actctgggta acctgcagca ggtcgccaac     960
   ggcgaaacct gtccgaacgc catcgtctaa                                     990
<210> 2
   <211> 1200
   <212> DNA
   <213> Artificial Sequence
<220>
   <223> Artificial sequence
<400> 2
<210> 3
   <211> 30
   <212> DNA
   <213> Artificial Sequence
<220>
   <223> Artificial sequence
<400> 3
   tagaggatcc caagcgtgcg cggtgaaccg 30
<210> 4
   <211> 47
   <212> DNA
   <213> Artificial Sequence
<220>
   <223> Artificial sequence
<400> 4
   gaggagcaca aaagggaaag gcgaagactt ttctccagtg attatac 47
<210> 5
   <211> 1199
   <212> DNA
   <213> Artificial Sequence
<220>
   <223> Artificial sequence
<400> 5
   cgcctttccc ttttgtgctc ctctcccggg gggagcacat tcagataatc cccacagatc      60
   cctgctgcga taccgttaca ctggcttggt tttattagtt atatgattgt tttggagtga     120
   aaatgaacaa atttgcggcg cttctggcgg caggtatgct gctgtccggc tgtgtctata     180
   atagtaaggt gtccaccggt gcggaacagc tgcagcatca tcgtttcgtg ctgaccagcg     240
   tcaacggcca ggcggtcaac gccagcgacc ggccgctgga gctgagcttc ggtgagaaga     300
   tggctattac cggcaagatg tatgtatccg gcaatatgtg caacggcttt agcggggaag     360
   gtaaagtgtc ggacggcgag ctgaaggtca aatcgctggc gatgacccgg atgctgtgcc     420
   acgacgccca gctcaatacc ctggatgcga cgatcgacaa gatgctgcgc gagggtgcgc     480
   aggtcgatct gacggaaaac cagttgacgc tggcgaccgc cgaccagacg ctggtctata     540
   agctcgccga cctgatgcac tagccggcgt tgaggtgccg ctgacgctgc cccgcgacgg     600
   ggccgctgtt agtagccgca gctgccaccc gccagcgcct gctcgctgca gcgtttgccg     660
   ttcggcagcg cgcacatgcc aatcgccgaa ccatcgagct gacgagccac cgataacgag     720
   ccgcctatca tggcgcagtt ggcctgaccg gcgtcgctca tcgccgcccg cattcccggc     780
   gtgacgtgcg ccgccgtggc ctgctgaacg ggttcactac tgcacgcgga cagcaacagc     840
   gccgcacatc ctactaacat cgcagctcgc attctctctc ccctcggaaa cgtcttaaaa     900
   aagcaaaccc cagaataata ggcagcgtgg cgggcggcgt cgagagggga agtacgtatt     960
   tatgcgcctc attaacattt tctagcaaat tttcgcctaa agcttgatct gcctcggcca    1020
   tgtcgcccgg cgcaggtggt tcatctcccg gcaggcagcc attttctccg cgaaccacgc    1080
   aaaatattga tctggtcacg ggtacccggc gcattgagga cacaaatgca aaaatggcgg    1140
   ggtcagcggt ttgctaaact accccttata taattacagg gcgcgtcgcg gtttcacgc     1199
<210> 6
   <211> 47
   <212> DNA
   <213> Artificial Sequence
<220>
   <223> Artificial sequence
<400> 6
   gtataatcac tggagaaaag tcttcgcctt tcccttttgt gctcctc 47
<210> 7
   <211> 31
   <212> DNA
   <213> Artificial Sequence
<220>
   <223> Artificial sequence
<400> 7
   atcgcggccg cgcgtgaaac cgcgacgcgc c 31
<210> 8
   <211> 2399
   <212> DNA
   <213> Artificial Sequence
<220>
   <223> Artificial sequence
<400> 8
   caagcgtgcg cggtgaaccg ggagagggat cgctggccgg cagtttgctc aggcaggcgc      60
   tgttgatctc cagctggcca atatgcagcc gccagcggct gggacgcgag agacgggcat     120
   cggtcacccg ggcgatttca cagtcgccca ccagataacg cagatcgggg atcagcaggg     180
   ccgaccgcgt caggcgcggg ctctcctgca aagagatacg cgtgcccacg ggcagccaga     240
   tgcccgccag cgtcggcacc cagtgggtta gcgtcaacag cagggttagc ggcaataaca     300
   ccagaactaa caccagcgcg atggcggctt tatatttacc cttcatgggc agttaatatc     360
   ctgattcaac ataagtaaaa gccgaaaggc gtccattgtg acacgttcga ccagtgagtg     420
   aaagtttacg gcctgttaaa gcatagttgc cagccggact cgcggcgcga cgttcggcca     480
   ttatcattta actgttgttt aagtcgcccc tgccacactc cagccagacg ggaatagctt     540
   gcgggagagg cggtgtcgtt aattatctcg ctcatagaga gcgcacagga ccactatcca     600
   tgggtattgc tgattgtttt tctgcttacc ttcactaaat cctgcgcatt ggtctcgctg     660
   gcaatccccg gcacctccgg cctgctgctg ctggggacat tcgcttccgc cagcctcgga     720
   catttcctgt taatgtggtc cagcgccagc ctcggcgcca tcggcggatt ctggctatcg     780
   tggcggctgg gcattcgcta ccgtcatcgc ctcacccatc tacgctggct gaccgccgag     840
   cgtctggccc gcagccgcct cttctttcag cgctatggcc cgtgggctat ctttttcagc     900
   cgctttctct ctcccctgag ggctacgctg cccttcgtta gcggcgccag cagtctgccg     960
   ctgtggtcgt ttcagctggc taacgtcagc tccggtctgc tgtggccgct tctgctgctc    1020
   gcccccggcg ctttcagcct cagtttgtgg tgaaaaaact ttgtctttca aagagattcc    1080
   gcaagtccgc gatatgctct agaattagga ttagcaccct ctcattaaac tattttttaa 1140
   taattgtacg attattttaa atatgctacc gtgacggtat aatcactgga gaaaagtctt 1200
   cgcctttccc ttttgtgctc ctctcccggg gggagcacat tcagataatc cccacagatc 1260
   cctgctgcga taccgttaca ctggcttggt tttattagtt atatgattgt tttggagtga 1320
   aaatgaacaa atttgcggcg cttctggcgg caggtatgct gctgtccggc tgtgtctata 1380
   atagtaaggt gtccaccggt gcggaacagc tgcagcatca tcgtttcgtg ctgaccagcg 1440
   tcaacggcca ggcggtcaac gccagcgacc ggccgctgga gctgagcttc ggtgagaaga 1500
   tggctattac cggcaagatg tatgtatccg gcaatatgtg caacggcttt agcggggaag 1560
   gtaaagtgtc ggacggcgag ctgaaggtca aatcgctggc gatgacccgg atgctgtgcc 1620
   acgacgccca gctcaatacc ctggatgcga cgatcgacaa gatgctgcgc gagggtgcgc 1680
   aggtcgatct gacggaaaac cagttgacgc tggcgaccgc cgaccagacg ctggtctata 1740
   agctcgccga cctgatgcac tagccggcgt tgaggtgccg ctgacgctgc cccgcgacgg 1800
   ggccgctgtt agtagccgca gctgccaccc gccagcgcct gctcgctgca gcgtttgccg 1860
   ttcggcagcg cgcacatgcc aatcgccgaa ccatcgagct gacgagccac cgataacgag 1920
   ccgcctatca tggcgcagtt ggcctgaccg gcgtcgctca tcgccgcccg cattcccggc 1980
   gtgacgtgcg ccgccgtggc ctgctgaacg ggttcactac tgcacgcgga cagcaacagc 2040
   gccgcacatc ctactaacat cgcagctcgc attctctctc ccctcggaaa cgtcttaaaa 2100
   aagcaaaccc cagaataata ggcagcgtgg cgggcggcgt cgagagggga agtacgtatt 2160
   tatgcgcctc attaacattt tctagcaaat tttcgcctaa agcttgatct gcctcggcca 2220
   tgtcgcccgg cgcaggtggt tcatctcccg gcaggcagcc attttctccg cgaaccacgc 2280
   aaaatattga tctggtcacg ggtacccggc gcattgagga cacaaatgca aaaatggcgg        2340
   ggtcagcggt ttgctaaact accccttata taattacagg gcgcgtcgcg gtttcacgc         2399
<210> 9
   <211> 939
   <212> DNA
   <213> Klebsiella pneumoniae
<400> 9
   atgaaagttg cagtccttgg cgctgccggt ggtatcggcc aggcgcttgc cctactactt 60
   aagacccaac tgccttcagg ttcagagctc tcgttgtacg acatcgctcc ggttacgccg 120
   ggcgtggcgg tagatctaag tcatatcccc acagatgtaa aaattaaagg attttccggt 180
   gaagacgcta ctccggcgct ggaaggcgcg gatgtagtgc tgatctccgc gggcgtggcg 240
   cgtaagcccg gcatggatcg ttccgacctg tttaatgtga atgcgggtat cgtgaagaac 300
   ctcgtgcagc agattgccaa aacctgcccg caggcctgca tcggcattat caccaacccg 360
   gtgaatacca ccgtggctat cgccgccgaa gtactgaaaa aagccggcgt gtacgataaa 420
   aacaaactgt tcggcgttac cacgctggac atcatccgtt ccaatacctt tgtggcggag 480
   ctgaaaggta aatcggcaac cgaggtggaa gtcccggtca ttggtggtca ctccggggtc 540
   accattctgc ctttactgtc gcagatcccc ggcgtcagct ttagcgatca ggaaattgcc 600
   gacctgacta aacgtattca gaacgccggt actgaagtcg tggaagcgaa agcgggcggc 660
   gggtcggcga ccttgtcgat gggccaggcg gctgcccgtt ttggtctctc tctggttcgc 720
   gccatgcagg gggaaaaagg cgtggtggag tgcgcctacg tggaaggcga cggccactat 780
   gcgcgtttct tctcccagcc gctgctgctg gggaaaaacg gcgtggaaga gcgacagtct 840
   atcggcaaac tcagcgcctt tgaacagcag gcgctggggg gcatgctgga caccctgaag 900
   aaagatatcg cgctcggcga agacttcgtc aacaagtaa 939
<210> 10
   <211> 1010
   <212> DNA
   <213> Artificial Sequence
<220>
   <223> Artificial sequence
<400> 10
   cgcgttagta aaagaacgta tagacggcgt aaatgacact aagtgttaaa tagttgttgt 60
   gccagaaggc gggagaggca aaagaaaggg gagtcgccct ctgacggtag ccagagggga 120
   attactcaga gttcctgctc aaacaggacc agaatggcgt cgtgcagatc tttcaccgta 180
   aaaccgcgcg caggcgtggt aaagatggtg tcatccccgg cgatactgcc aagaatgcct 240
   tccgctttgc ccagcgagtc gagaagacgg gcgatcagct gtgcggcgcc ggggctggta 300
   tgaatcacca ctacggcatc gttatagtcg atatccagca ccaaattctt cagcgggctg 360
   gaggtggtcg gtaccccaag ctcggcaggc aagcaataga ccatttccat tttggcgtta 420
   cgggtacgca ccgcgccaaa tttggtgagc atgcgggaaa ccttcgattg gttgatattc 480
   tcgaagccct cttcctgcag cgcctggacg atctctccct gcgagctgaa tttctcttct 540
   ttaagcagcg ctttaaacgc tttaaccagt tcttcttgtt ttgccgaact tcgcataagt 600
   cacccagatt atggtggtaa aatcaacatc attatgcata accatgaatt tttatgcaaa 660
   taatccgcga aaggataggt caataatgtt atgaaggggg catattttag caaaaaaacc 720
   gtataaggaa catgttctca tcacaacgct taaagccatg taaaagtaaa tgaaatgtta 780
   ttgagttggt gttgttttgc gcaactggct gggtgtattg taaccaatag tcgatccggt 840
   acttcgcttt gctggatggt cagtcaggaa gcataaagtg cgccgctgtt ctattgctac 900
   gcacaataat gcattcttca gccataatca ttgtaattgt tcatcttgtg aattaaggtc 960
   caggcgacgg aataacgaaa gactaaatta accataataa ggagtttagg 1010
<210> 11
   <211> 33
   <212> DNA
   <213> Artificial Sequence
<220>
   <223> Artificial sequence
<400> 11
   gtcgaccgcg ttagtaaaag aacgtataga cgg 33
<210> 12
   <211> 39
   <212> DNA
   <213> Artificial Sequence
<220>
   <223> Artificial sequence
<400> 12
   gctcttctgt tagggcgaga cctaaactcc ttattatgg 39
<210> 13
   <211> 999
   <212> DNA
   <213> Artificial Sequence
<220>
   <223> Artificial sequence
<400> 13
   tctcgcccta acagaagagc cggaaccgct gttccggctt tttattgtct agcattcatg 60
   gaaaccgggt ttctggtcag attcccggcc ggtccggtat ttagcgatta acgtggctcc 120
   ccaaacgggg agccgtttag ttggtggccg ggtattcctg gacggcgata tggagcgtga 180
   ttttcttatc atcacgcatg atgaccaccg gaatttccga tcccgggcgg atctcggcca 240
   cctgatccat cgtctccagc gccgagacgg cgggtttgtc attcacggag atgattacgt 300
   cgttggcgcg gatcccggct tgagccgctg gaccgtccgg ggccacatcg ttaacgacga 360
   tcccctgaat ctgatcgatc cctccgccct gcgcatgcag cggggcgatc tcccggccgc 420
   taatgccgat atagccgcgg atcacccggc catcgcggat caatttatcc ataattttgg 480
   tcgctaactg gaacgggatc gcaaagccaa tgccttccgg cgtttcgccg tcattgctct 540
   tgtcaaagga gagggtgtta atccccatca gctcgccgag ggagttcacc agcgccccgc 600
   cggagttacc gtggttgatc gaggcgtcag tctgcaggaa gttctggcgg ccggtcggat 660
   tgaggccaat gcgccctgtg gcgctgataa tcccctgggt gatggtctgc cccaggttat 720
   aagggttgcc aatcgccagc accacgtcgc cgatgtgcgg cgtgcgtttc ggattgatcg 780
   ggatcaccgg cagcccgccg gtagcattga tcttgagcac ggcgagatcg gtgagggaat 840
   cggatcctac cagcagcgct tcgaagacgc ggccgtcctg cagggcgacg ataatctgat 900
   cggcatcgtt gataacatgc ttgttggtca ggatatagcc gcgctgatcc atgatcactc 960
   cggaacccag cgtcagctga ttatggctgg tgctgttca 999
<210> 14
   <211> 39
   <212> DNA
   <213> Artificial Sequence
<220>
   <223> Artificial sequence
<400> 14
   ccataataag gagtttaggt ctcgccctaa cagaagagc 39
<210> 15
   <211> 34
   <212> DNA
   <213> Artificial Sequence
<220>
   <223> Artificial sequence
<400> 15
   gcggccgctg aacagcacca gccataatca gctg 34
<210> 16
   <211> 2009
   <212> DNA
   <213> Artificial Sequence
<220>
   <223> Artificial sequence
<400> 16
   cgcgttagta aaagaacgta tagacggcgt aaatgacact aagtgttaaa tagttgttgt 60
   gccagaaggc gggagaggca aaagaaaggg gagtcgccct ctgacggtag ccagagggga 120
   attactcaga gttcctgctc aaacaggacc agaatggcgt cgtgcagatc tttcaccgta 180
   aaaccgcgcg caggcgtggt aaagatggtg tcatccccgg cgatactgcc aagaatgcct 240
   tccgctttgc ccagcgagtc gagaagacgg gcgatcagct gtgcggcgcc ggggctggta 300
   tgaatcacca ctacggcatc gttatagtcg atatccagca ccaaattctt cagcgggctg 360
   gaggtggtcg gtaccccaag ctcggcaggc aagcaataga ccatttccat tttggcgtta 420
   cgggtacgca ccgcgccaaa tttggtgagc atgcgggaaa ccttcgattg gttgatattc 480
   tcgaagccct cttcctgcag cgcctggacg atctctccct gcgagctgaa tttctcttct 540
   ttaagcagcg ctttaaacgc tttaaccagt tcttcttgtt ttgccgaact tcgcataagt 600
   cacccagatt atggtggtaa aatcaacatc attatgcata accatgaatt tttatgcaaa 660
   taatccgcga aaggataggt caataatgtt atgaaggggg catattttag caaaaaaacc 720
   gtataaggaa catgttctca tcacaacgct taaagccatg taaaagtaaa tgaaatgtta 780
   ttgagttggt gttgttttgc gcaactggct gggtgtattg taaccaatag tcgatccggt 840
   acttcgcttt gctggatggt cagtcaggaa gcataaagtg cgccgctgtt ctattgctac 900
   gcacaataat gcattcttca gccataatca ttgtaattgt tcatcttgtg aattaaggtc 960
   caggcgacgg aataacgaaa gactaaatta accataataa ggagtttagg tctcgcccta 1020
   acagaagagc cggaaccgct gttccggctt tttattgtct agcattcatg gaaaccgggt 1080
   ttctggtcag attcccggcc ggtccggtat ttagcgatta acgtggctcc ccaaacgggg 1140
   agccgtttag ttggtggccg ggtattcctg gacggcgata tggagcgtga ttttcttatc 1200
   atcacgcatg atgaccaccg gaatttccga tcccgggcgg atctcggcca cctgatccat 1260
   cgtctccagc gccgagacgg cgggtttgtc attcacggag atgattacgt cgttggcgcg 1320
   gatcccggct tgagccgctg gaccgtccgg ggccacatcg ttaacgacga tcccctgaat 1380
   ctgatcgatc cctccgccct gcgcatgcag cggggcgatc tcccggccgc taatgccgat 1440
   atagccgcgg atcacccggc catcgcggat caatttatcc ataattttgg tcgctaactg 1500
   gaacgggatc gcaaagccaa tgccttccgg cgtttcgccg tcattgctct tgtcaaagga 1560
   gagggtgtta atccccatca gctcgccgag ggagttcacc agcgccccgc cggagttacc 1620
   gtggttgatc gaggcgtcag tctgcaggaa gttctggcgg ccggtcggat tgaggccaat 1680
   gcgccctgtg gcgctgataa tcccctgggt gatggtctgc cccaggttat aagggttgcc 1740
   aatcgccagc accacgtcgc cgatgtgcgg cgtgcgtttc ggattgatcg ggatcaccgg 1800
   cagcccgccg gtagcattga tcttgagcac ggcgagatcg gtgagggaat cggatcctac 1860
   cagcagcgct tcgaagacgc ggccgtcctg cagggcgacg ataatctgat cggcatcgtt 1920
   gataacatgc ttgttggtca ggatatagcc gcgctgatcc atgatcactc cggaacccag 1980
   cgtcagctga ttatggctgg tgctgttca 2009
<210> 17
   <211> 1647
   <212> DNA
   <213> Klebsiella pneumoniae
<400> 17
   atgtcgaata aaccgtttgt ttaccaggat ccttttccgc tcaaaaagga tgacaccgag      60
   tattatctcc tcagcagtga ttacgtctcc gtcgccgaat tcgccggcca ggaagtgcta     120
   aaagttgacc ctcaggcgct gacccttctg gcgcagcatg ctttccacga cgcctccttt     180
   atgcttcgcc ctgcgcatca gcagcaggtg gcggatatcc ttaacgatcc ggaagccagc     240
   gaaaacgata aatacgtcgc gctgcagttc ctgcgcaact cggatatcgc cgccaaaggt     300
   attctgccca cctgccagga caccggcacc gcgatcatca tgggtaaaaa aggccagcgc     360
   gtgtggaccg gcggcggcga tgaagccgcc ctggcgcagg gggtgtacaa cacctatatc     420
   caggacaacc tgcgctactc gcagaacgcg ccgctggata tgtataaaga ggttaacact     480
   ggcaccaacc tgccggcgca gatcgatctc tatgctaccg atggcgatga gtacaaattc     540
   ctctgcatcg ccaagggcgg cggctcggcg aacaaaacct atctgtacca ggaaaccaaa     600
   gcgctgatca ccccggcgaa gctgaaaaat tacctcgtcg agaagatgcg caccctcggt     660
   actgccgcct gtccaccgta ccatatcgcc ttcgtgatcg gcggtacctc ggcggaagcg     720
   acgctgaaga ccgtgaagct ggcgtccacc aaatactacg atggcctgcc gaccgaaggt     780
   aacgagcacg gccaggcgtt ccgcgacgtg cagatggagc aggagctgct gctggaggcg     840
   cagaaccttg gtctcggcgc gcagttcggc ggcaaatact ttgcccacga tatccgcgtg     900
   atccgtctgc cgcgtcacgg cgcttcctgc ccggtaggga tgggggtttc ctgttccgcc     960
   gaccgcaaca tcaaagccaa gattaaccgc gacggcatct ggatcgagaa gctggagagc    1020
   aacccgggga aatatatccc tgagcatctg cgccaggccg gggaaggcga agcggtgaag    1080
   gtaaatctta accagccgat gagcgagatc ctggcgcagc tgtcgcagta tccggtgtcc    1140
   acccgcctgt cgctgagcgg gactatcatc gtcgcgcgcg atatcgccca cgctaagctg    1200
   aaggagctta tcgacaacgg cgaggcgctg ccgcagtacg tcaaagatca cccgatctac    1260
   tacgcaggtc cggccaagac gccggatggt tacgcctccg gctctctggg gccgaccacc    1320
   gcaggccgca tggactctta cgttgatctg ctgcagtccc atggggcgag caagatcatg    1380
   ctggcgaaag gcaaccgcag ccagcaggtc accgacgcct gccacaaaca cggcggcttc    1440
   tacctcggca gcatcggcgg cccggcggca gtgctggccc agcagagcat caagagcctc    1500
   gagtgcgtgg cctatccgga gctgggtatg gaggctatct ggaaaattga agtggaggat    1560
   ttcccggcct ttatcctcgt tgatgataaa ggcaatgact tcttccagca gatccagacc    1620
   tcgcagtgcg cccgctgcgt gaagtaa                                        1647
<210> 18
   <211> 958
   <212> DNA
   <213> Artificial Sequence
<220>
   <223> Artificial sequence
<400> 18
   tttcagcaaa caggaacatc gcttcgcctg ggttcagctt caccacgttc agcagcagcg 60
   gggagaagag gccgctgtcg tccggataga attccgcgat caggcggata gtctgccacg 120
   gttcgccttg ttcgcgggcc agtacgtcgc gcagcacctg cagcgccttg gccttttcct 180
   caccctgcat atttagcagg ctggcaaaca gctggctcag gtgagtcgcg tccggctgct 240
   gcaggaaggc gccgattgcc ggatgcgcgc tagccaccgg ctgcagcagg gtgacaatct 300
   cgctgaactc gcgaaatgca ttcatcgcca ggaacggcgt cagggcaaaa accagctccg 360
   gtttgtggtt cggatctttg tagttacgct cggcggcgga gagcgggatg ccggcggcgt 420
   tctcgcgggc gaaaccttct tccgaggcct gcttgttggg atgcacctga attgagagcg 480
   gctgagcggc gcacagtacc ttaaacagga acggcagttc accaaagcgg gcggcaacct 540
   tgtcgccgag cagagcggct ttatccgcgt caatcacctc gcgcagtgaa cgcggctggc 600
   cgtcggccgc caggatctgc gagctgctct tcggatgcgc ccccatccac agctctgcca 660
   tcggcagatt gtccgggttg gcgatgccat acagttcagt taaggcggtg tggcttcccc 720
   atgcatagtt ctgcacggcg ttaatcagct tttgcattct caatccctgt ttcaaacaaa 780
   gtaaatttta tcgccctatt aaaccagcaa acggtggaga agcaactgct gaaagaaaaa 840
   gtcgtccttg tctcagtttt tgttaaaaaa ttgtgtaaga ttgttcgact cgcttttcta 900
   caggcacatg gagtccctgc ctgaaacata atcagccaaa gcagtaagtg agagtaca 958
<210> 19
   <211> 38
   <212> DNA
   <213> Artificial Sequence
<220>
   <223> Artificial sequence
<400> 19
   ggatcctttc agcaaacagg aacatcgctt cgcctggg 38
<210> 20
   <211> 45
   <212> DNA
   <213> Artificial Sequence
<220>
   <223> Artificial sequence
<400> 20
   ctaacgaaaa caggtttgtc atctgtactc tcacttactg ctttg 45
<210> 21
   <211> 1107
   <212> DNA
   <213> Artificial Sequence
<220>
   <223> Artificial sequence
<400> 21
   gatgacaaac ctgttttcgt tagggtgagc gcctttctgc cgggcggcgg ctttcgcctt 60
   acccggccta cgaaaaacag caatatcaat aagttatgca gttctctgta ggcccgcgca 120
   agcgcagcgc cgccgggcaa tcccgacgat tgcattttgt cagcggtctg ggccgcccct 180
   cggggcggct ttttttatgt cgcttcgcgg cacgaaccac caaaacaact tatcaatctg 240
   gccaatactt agtcttttaa gcaggtgagc tttgtcgcta tgacgcactt ttcatccaag 300
   gagaaagtaa tgacaacgca tcgcagtgaa aaagactcga tgggcgccat cgaggtcccg 360
   gccgataaac tctggggcgc ccagacccag cgctcgctgg agcatttccg catctcgacc 420
   gaaaaaatgc ccggggagct gatatacgcc ctggcgctga ccaagcgggc ggcggcgaag 480
   gttaaccagg atctcggact gttgacggcg gagaaagccg gggcgatcgt cgccgccgct 540
   gacgaagtgc tggccggaaa gcacgcgcag gagttccccc tggctatctg gcagaccggc 600
   tccggcaccc aaagcaatat gaatatgaac gaggtgctgg ccaaccgcgc cagtgaactg 660
   ctcggcggag aacgggggat ggcgcgaaaa attcatccca atgacgatgt gaataagagc 720
   cagagctcaa atgatgtatt ccccaccgcc atgcacgtcg cggcgctgat tgccctgcgg 780
   gaaaaagtga tcccgtcgct gcaggcgctg cgggcgacgc tcaatgaaaa agcggtggcc 840
   ttcagggata tcgtcaagat cggccgcacc catctgcagg acgccacgcc gctcacctta 900
   ggccaggaga tctccggctg ggtggcgatg ctggaccata gcctgaagca cattgaagcc 960
   agtcagccgc atcttgccga gctggcgctg ggcgggacgg cggtaggaac cgggctgaat 1020
   acccatccgg aatatgccgt acgggtggcg gccgagctgg cgtcgctcag cggacagcca 1080
   tttgtcaccg cgccgaacaa attcgaa 1107
<210> 22
   <211> 45
   <212> DNA
   <213> Artificial Sequence
<220>
   <223> Artificial sequence
<400> 22
   caaagcagta agtgagagta cagatgacaa acctgttttc gttag 45
<210> 23
   <211> 37
   <212> DNA
   <213> Artificial Sequence
<220>
   <223> Artificial sequence
<400> 23
   gcggccgctt cgaatttgtt cggcgcggtg acaaatg 37
<210> 24
   <211> 2065
   <212> DNA
   <213> Artificial Sequence
<220>
   <223> Artificial sequence
<400> 24
   tttcagcaaa caggaacatc gcttcgcctg ggttcagctt caccacgttc agcagcagcg 60
   gggagaagag gccgctgtcg tccggataga attccgcgat caggcggata gtctgccacg 120
   gttcgccttg ttcgcgggcc agtacgtcgc gcagcacctg cagcgccttg gccttttcct 180
   caccctgcat atttagcagg ctggcaaaca gctggctcag gtgagtcgcg tccggctgct 240
   gcaggaaggc gccgattgcc ggatgcgcgc tagccaccgg ctgcagcagg gtgacaatct 300
   cgctgaactc gcgaaatgca ttcatcgcca ggaacggcgt cagggcaaaa accagctccg 360
   gtttgtggtt cggatctttg tagttacgct cggcggcgga gagcgggatg ccggcggcgt 420
   tctcgcgggc gaaaccttct tccgaggcct gcttgttggg atgcacctga attgagagcg 480
   gctgagcggc gcacagtacc ttaaacagga acggcagttc accaaagcgg gcggcaacct 540
   tgtcgccgag cagagcggct ttatccgcgt caatcacctc gcgcagtgaa cgcggctggc 600
   cgtcggccgc caggatctgc gagctgctct tcggatgcgc ccccatccac agctctgcca 660
   tcggcagatt gtccgggttg gcgatgccat acagttcagt taaggcggtg tggcttcccc 720
   atgcatagtt ctgcacggcg ttaatcagct tttgcattct caatccctgt ttcaaacaaa 780
   gtaaatttta tcgccctatt aaaccagcaa acggtggaga agcaactgct gaaagaaaaa 840
   gtcgtccttg tctcagtttt tgttaaaaaa ttgtgtaaga ttgttcgact cgcttttcta 900
   caggcacatg gagtccctgc ctgaaacata atcagccaaa gcagtaagtg agagtacaga 960
   tgacaaacct gttttcgtta gggtgagcgc ctttctgccg ggcggcggct ttcgccttac 1020
   ccggcctacg aaaaacagca atatcaataa gttatgcagt tctctgtagg cccgcgcaag 1080
   cgcagcgccg ccgggcaatc ccgacgattg cattttgtca gcggtctggg ccgcccctcg 1140
   gggcggcttt ttttatgtcg cttcgcggca cgaaccacca aaacaactta tcaatctggc 1200
   caatacttag tcttttaagc aggtgagctt tgtcgctatg acgcactttt catccaagga 1260
   gaaagtaatg acaacgcatc gcagtgaaaa agactcgatg ggcgccatcg aggtcccggc 1320
   cgataaactc tggggcgccc agacccagcg ctcgctggag catttccgca tctcgaccga      1380
   aaaaatgccc ggggagctga tatacgccct ggcgctgacc aagcgggcgg cggcgaaggt      1440
   taaccaggat ctcggactgt tgacggcgga gaaagccggg gcgatcgtcg ccgccgctga      1500
   cgaagtgctg gccggaaagc acgcgcagga gttccccctg gctatctggc agaccggctc      1560
   cggcacccaa agcaatatga atatgaacga ggtgctggcc aaccgcgcca gtgaactgct      1620
   cggcggagaa cgggggatgg cgcgaaaaat tcatcccaat gacgatgtga ataagagcca      1680
   gagctcaaat gatgtattcc ccaccgccat gcacgtcgcg gcgctgattg ccctgcggga      1740
   aaaagtgatc ccgtcgctgc aggcgctgcg ggcgacgctc aatgaaaaag cggtggcctt      1800
   cagggatatc gtcaagatcg gccgcaccca tctgcaggac gccacgccgc tcaccttagg      1860
   ccaggagatc tccggctggg tggcgatgct ggaccatagc ctgaagcaca ttgaagccag      1920
   tcagccgcat cttgccgagc tggcgctggg cgggacggcg gtaggaaccg ggctgaatac      1980
   ccatccggaa tatgccgtac gggtggcggc cgagctggcg tcgctcagcg gacagccatt      2040
   tgtcaccgcg ccgaacaaat tcgaa                                            2065